# EUROPEAN PATENT APPLICATION

(11) **EP 4 289 469 A1**
(43) Date of publication of application: **13.12.2023**
(21) Application number: 22177702.2
(22) Date of filing: 08.06.2022
(51) Int. Cl.: A61N 1/375, A61N 1/372, A61N 1/05, A61M 31/00, A61M 37/00

(54) **HEADER ASSEMBLY FOR AN IMPLANTABLE INTRACARDIAC DEVICE AND RESPECTIVE INTRACARDIAC DEVICE**

(71) Applicant: BIOTRONIK SE & Co. KG, 12359 Berlin (DE)
(72) Inventor: Taff, Brian M., Portland, 97217 (US)
(74) Representative: Biotronik Corporate Services SE

(57) **Abstract**

The invention refers to an implantable intracardiac device comprising a header assembly, wherein the header assembly comprises an electrically insulating sub-assembly (2.1, 2.2, 2.3, 2.4) and an electrode, wherein the electrode comprises a distal head section (1.2) and a proximal shaft section (1.1) and is accommodated within a through hole (2.5) of the insulating sub-assembly (2.1, 2.2, 2.3, 2.4), wherein the insulating sub-assembly (2.1, 2.2, 2.3, 2.4) comprises fixation means (2.2, 2.2.1, 2.2.2, 2.2.3) protruding from its distal end and a medicament eluting depot (2.4) forming at least one end face section of a distal end face of the insulating sub-assembly (2.1, 2.2, 2.3, 2.4) and/or at least one inner surface section of an inner surface of the through hole (2.5) of the insulating sub-assembly (2.1, 2.2, 2.3, 2.4), wherein the at least one end face section and/or the at least one inner surface section is located adjacent to and abuts the head section (1.2) of the electrode, wherein the head section of the electrode comprises open porosity (5.2) within at least part of its volume configured to lead at least part of the medicament eluted from the medicament eluting depot (2.4) through the head section (1.2) and to the head section's distal end. The invention further refers to a respective implantable intracardiac device.

## Description

The present invention refers to an implantable intracardiac device, such as an implantable intracardiac pacemaker, and a header assembly therefor.

Active or passive implantable intracardiac devices, for example implantable intracardiac pacemakers (also known as implantable leadless pacemakers - ILPs), are well known miniaturized medical devices which are entirely implanted into a heart's ventricle or atrium. Intracardiac pacemakers apply electrical stimulation in the form of pulses to the heart to generate a physiologically appropriate heartrate while cardioverter defibrillators apply electrical stimulation in the form of shocks to restore a more normal heart rhythm. Alternative or additional functions of intracardiac devices comprise providing other electrical or electromagnetic signals to the heart or its surrounding tissue, sensing electrical or electromagnetic signals or other physiological parameters of the heart and/or its surrounding tissue.

Leadless pacemakers largely aim to provide support as has been delivered for bradycardia management in traditional, pocket based leaded IPGs through devices sized at ~10% of the total volume of legacy formats. While this miniaturization permits the placement of leadless implants within the blood volume of a patient's heart and provides reduced risk for infection through the elimination of leaded interfacing with the myocardium, an ILP has a small battery capacity due to the highly restricted device size. The requirements in terms of operational longevity are, however, the same. It is, therefore, paramount to minimize current draws from the battery during operation. That is to be achieved by ensuring optimal contact between the ILP electrode tip and the heart tissue. Nevertheless, implantation of an ILP to a patient's heart may trigger immune and/or foreign-body response at the device-tissue-interface and the anchoring positions. Said responses involve, e.g. swelling of the tissue, which can lower the ability to electrically stimulate conduction pathways within the myocardium. This is especially problematic when occurring at the pacing and sensing site as it is linked to losses in pacing and sensing efficiency.

To suppress unwanted foreign-body and immune response of the patient, steroids and other anti-inflammatory drugs are administered in proximity to a pacemaker's sensing and/or pacing tissue interface. Furthermore, the fixation areas of the ILP have been separated locally from the electrode to minimize the interplay of responses caused by anchoring and pacing/sensing. Such separation is distinct from traditional "active" lead designs which pace/sense at the exact same location where mechanical anchoring occurs and causes scarring. The separation presumably means that the pacing/sensing can more readily occur without having to overcome more sizeable volumes of damaged patient tissue to reach conduction pathways within the myocardium.

Document US 2019/0083779 A1 discloses an ILP which comprises a housing, an electrically insulative distal member, which is coupled directly to the housing and a tissue piercing, cylindrical distal dart electrode that extends away from the housing. The ILP is coupled to the heart's tissue using fixation members, e.g. tines, that extend from the distal end of the housing. The distal tips of the tines penetrate the heart tissue to a limited depth before elastically curving back proximally into the normally curved position. Within the electrode there is central member being a drug reservoir in the form of a steroid impregnated polymer member, which allows to elute a steroid through the exposed tip electrode portion. However, such ILP has a very small volume for the steroid as its reservoir is accommodated within the electrode thereby limiting the time period in which the drug is administered.

Known administration techniques of medicaments, e.g. anti-inflammatory drugs, have stationed drug-elution elements close to the electrode and in locations where they can both make direct contact with the cardiac tissue. While these approaches have certainly improved said administration to decrease immune and foreign-body responses, they still lack the ability to deliver the drug(s) at the exact position as sensing/pacing or have only a very limited volume for the medicaments as in the above- mentioned example where a steroid impregnated polymer is located within the electrode.

Accordingly, there is the need for implantable intracardiac devices and respective header assemblies that allow to elute a medicament at the site where sensing/pacing occurs and that realize a long-lasting medicament treatment.

The above problem is solved by an implantable intracardiac device comprising a header assembly comprising the features of claim 1.

In particular, the above problem is solved by a header assembly, wherein the header assembly comprises an electrically insulating sub-assembly and an electrode, wherein the electrode comprises a distal head section and a proximal shaft section and is accommodated within a through hole of the insulating module, wherein the insulating sub-assembly comprises fixation means protruding from its distal end and a medicament eluting depot forming at least one end face section of a distal end face of the insulating sub-assembly and/or at least one inner surface section of an inner surface of the through hole of the insulating sub-assembly, wherein the at least one end face section and/or the at least one inner surface section is located adjacent to and abuts the head section of the electrode, wherein the head section of the electrode comprises open porosity within at least part of its volume configured to lead at least part of the medicament eluted from the medicament eluting depot through the head section and to the head section's distal end.

The implantable intracardiac device (in the following short: ID) may be, for example, an implantable intracardiac pacemaker (also known as leadless pacemaker) which may apply electrical stimulation in the form of pulses to the heart to generate a physiologically appropriate heartrate or it may be a cardioverter defibrillator which applies electrical stimulation in the form of shocks to restore a more normal heart rhythm. In the latter case, the ID is a defibrillator or cardioverter, instead. Alternative or additional functions of intracardiac devices may comprise providing other electrical or electromagnetic signals to the heart or its surrounding tissue, sensing electrical or electromagnetic signals or other physiological parameters of the heart and/or its surrounding tissue. In case, the ID is focused on sensing electrical or electromagnetic signals it may be alternatively called a monitor or loop recorder. The ID may realize any combination of above functions. The implantation of the ID may comprise any fixation to the heart's tissue comprising the fixing within the atria and the ventricles of the heart or a fixing at the outer surface of the heart's tissue using fixation means that may be formed as small tines or other means.

The inventive ID usually further comprises a hermetically closed cylindrical housing and the header assembly located at the distal end of the housing. Further, the header assembly comprises an electrically insulating sub-assembly with an opening, within which the pin-shaped electrode is accommodated and projects through the insulating sub-assembly, i.e. through a respective through-going or complete opening of the insulating sub-assembly. The pin-shaped electrode comprises a proximal shaft section and a distal head section. The through hole may be a central opening located at and along a longitudinal axis of the ID housing and of the insulating sub-assembly. The longitudinal axis forms the axial direction of the ID and the header assembly. The insulating sub-assembly provides electrical isolation of the pin-shaped electrode with regard to the ID housing.

The cylindrical housing comprises the electronics module having a processor, an energy source (e.g. a battery or coil for wireless charging) and, if applicable, a communication component such as an antenna (or inductive coil). The processor may be adapted to process signals/data determined from the patient's body or received from the surrounding environment and/or to produce signals for treatment of the patient's heart. Such signals may comprise electrical stimulation in the form of pulses to generate a physiologically appropriate heartrate, shocks for cardioversion or defibrillation to restore a more normal heart rhythm and/or other electrical or electromagnetic signals to the heart or its surrounding tissue. Such signals are transformed and transmitted by the electronic module and may be applied by the distal head section of the pin-shaped electrode to the heart or its surrounding tissue. The pin-shaped electrode with its proximal shaft section projects through a respective through-going opening of the housing (also referred to as feedthrough) into the housing and is electrically connected to the electronics module and the energy source located within the housing. The hermetically sealed housing may comprise electrically conducting material, e.g. titanium or stainless steel, and may function as a counter electrode.

The insulating sub-assembly further comprises the fixation means protruding from the electrically insulating sub-assembly in distal direction, for example comprising curved tines, for fixation of the ID to the selected tissue of the patient according to the treatment plan of the heath care practitioner (HCP), for example a ventricular wall of the patient's heart. Furthermore, the insulating sub-assembly comprises a medicament eluting depot, that contains a medicament comprising at least one medical substance, for example an anticoagulant and/or an anti-inflammatory substance. As described below in more detail the medicament may be released gradually into the surrounding tissue to heal or treat the damaged tissue after fixation of the intracardiac device with the header assembly at the target location within the patient's heart.

The insulating sub-assembly forms a distal end face and an inner surface of the through hole that extends along the axial direction of the insulating sub-assembly. The insulating sub-assembly may be one-piece or multi-piece, for example comprising at least two ring-shaped elements clamping and thereby holding the fixation means in place with a distance to the electrode. The medicament eluting depot forms at least a section of the insulating sub-assembly's distal end face and/or at least one inner surface section of the through hole. The medicament eluting depot may come in direct contact with the tissue and, therefore, allow direct administration of the stored medicament. Further, the head section of the electrode is positioned within the electrically insulating sub-assembly such that it is adjacent to and abuts the at least one end face section of the distal end face of the insulating sub-assembly and/or at least one inner surface section of the inner surface of the through hole of the insulating sub-assembly formed by the medicament eluting depot. Furthermore, the head section of the electrode has an open porosity at least in a part of the head section's volume or within its total volume, wherein the total volume or this volume part abuts the medicament eluting depot and allows the drug eluted from the medicament eluting depot to pass through. The network of passages internal to such porous electrodes provides conduits that enable leading of said medicament through the electrode directly to the tissue being in contact with and surrounding the electrode. In that way the medicament enters said part of the head section of the electrode and diffuses through the head section to the head section's distal end. Bringing the medicament eluting depot in direct contact with the porous head section of the electrode enables the electrode head section to elute the medicament at the electrode-tissue interface, i.e. at the exact position where sensing and/or pacing occurs. Additionally, locating the medicament eluting depot within the electrically insulating sub-assembly, provides a comparable large volume for the medicament thereby realizing a longer duration of action. Accordingly, the above-described header assembly outlines a means to optimize the device/tissue interface and improves device performance.

In one embodiment, the medicament eluting depot is doughnut-shaped and/or is at least partly exposed. The medicament eluting depot being exposed to the tissue in addition to being in contact with the porous head section of the electrode further increases the tissue contact area treated with the drug. The medicament eluting depot is at least partly exposed is to be understood as the medicament eluting depot is in the implanted state of the device in contact with the tissue at the implantation site and the surface of the medicament eluting depot is configured to allow the passage of the medicament from the depot to the tissue. Accordingly, a larger area of the surrounding tissue may be provided with the medicament as the insulating sub-assembly may additionally elute the medicament directly to the surrounding tissue. The application of a specific geometry such as a doughnut shape (may also be referred to as ring-shaped) to the medicament eluting depot may be beneficial as well, for example with regard to production costs. A doughnut-shaped depot comprises a through hole and thus an outer circumference and an inner circumference, wherein the outer circumference is larger than the inner circumference. Said form may also be regarded as a hollow cylinder. In one embodiment, the electrode is accommodated within the through-hole of the medicament eluting depot so that the depot circumvents the electrode and provides, thereby, a large abutting surface from which medicament is eluted and received by as well as led through the porous head section of the electrode. The medicament eluting depot, therefore, may abut a surface of the head section of the electrode that circumvents the longitudinal axis of the ID. In this way the medicament is eluted from the medicament eluting depot into the head section of the electrode not only in a small part of the head section, but from around the whole circumference of the head section.

In one embodiment, the head section of the electrode comprises or consists of a material manufactured by a powder metallurgical sintering process. In the process of sintering, powder is treated using heat to impart strength and integrity. A metallic powder of a pre-defined composition undergoes a heating process with a pre-defined temperature/pressure profile thereby creating the porous microscopic structure and the outer shape of the electrode head section, or portions thereof. In that way above-mentioned network of open pores acting as conduits is formed that allows the medicament to go through the material of the electrode. These material properties are regarded as open porosity, wherein porosity is the fraction of the volume of voids in the material of the electrode head section over the total volume of the electrode head section. The open porosity is the volume of voids that are connected to each other and the surroundings of the electrode head section. The open porosity of the porous part / the total head section of the electrode may be in the range 0.05 - 0.75. The manufacturing process using powder metallurgical sintering may involve packing the metal electrode material powder into a mold that consists of a negative of the desired shape of the electrode head section. It, furthermore, may involve accommodation, e.g. centering, of the proximal shaft section of the electrode in and/or at the powder prior to sintering. The sintering process may facilitate the mechanical and electrical connection between the, e.g. continuously solid (non-porous), proximal shaft section and the porous distal head section of the electrode.

In one embodiment, the material of the head section of the electrode comprises a platinum-iridium alloy comprising 70-92 weight percent (wt%) platinum and 8-30 wt% iridium, wherein the platinum and iridium alloy components together amount to 100 wt% at maximum. For example, the head section may consist of 90 wt% of platinum and 10 wt% of iridium. The material of the head section of the electrode may also comprise at least one additional material apart from platinum and iridium such as titanium or any other biocompatible metal. In one embodiment the material of the head section of the electrode may consist of 80 wt% platinum and 20 wt% iridium.

In one embodiment, the electrode head section comprises a proximal end face that abuts a distal end face of the medicament eluting depot. The proximal end face of the electrode head section and the distal end face of the medicament eluting depot extend transversally to the axial direction of the electrode. The medicament eluting depot may extend along the axial direction of the header assembly, and its length may correspond to the overall length of the header assembly in that direction. The electrode distal head section may, for example radially protrude from the proximal shaft section thereby forming the proximal end face. In other words, in this embodiment the diameter of the head section of the electrode is greater than the diameter of the proximal shaft section of the electrode. This is a simple arrangement of the medicament eluting depot and the electrode head section realizing an effective medicament elution through the electrode and to the electrode/tissue interface. In one embodiment the proximal end face having, e.g. a ring-shape, abuts a distal end face of the medicament eluting depot that has the above-described doughnut shape.

In one embodiment, the electrode head section has an exposed area at its distal end of 0.5 mm² to 2.5 mm². The size of the head section's exposed area defines the active area available for sensing/pacing. The exposed area may be at least partially flat and may have a surface normal parallel to the longitudinal axis of the ID, e.g. may have the form of a cuboid or a cylinder. Alternatively or additionally, the form of the exposed area of the electrode head section may be at least partially rounded, e.g. may be a semi-sphere or a shell surface of a cylinder. The cross section of the electrode head section (perpendicular to the longitudinal axis) may be centrosymmetric, e.g. may have a circular, rectangular- or quadratic shape. Additionally, the form of the electrode head section and/or its exposed part may have any combined form of the above-mentioned forms.

In one embodiment, the medicament eluting depot is a medicament-impregnated polymer member that provides medicament elution over time through the exposed electrode head section and, if applicable, also directly from the medicament eluting depot. The medicament eluting depot is typically formed using an organic polymer matrix, e.g. a silicone or polyurethane base that provides biocompatibility in combination with excellent drug loading and eluting capabilities. To guarantee continuous drug administration throughout the predetermined medicament administration time period of the device the medicament may have a pre-defined concentration and distribution within the polymer matrix. In one embodiment the medicament eluting depot is formed by a medicament-impregnated silicone member.

In one embodiment, the medicament comprises an anti-inflammatory drug, in particular a steroid, e.g. dexamethasone, and/or a non-steroidal anti-inflammatory drug which blocks the effects of the Cox-1 and/or Cox-2 enzymes, e.g. ibuprofen and/or naproxen. Electrical and/or mechanical stimulation of the heart's tissue may cause inflammatory and foreign-body response, for example in the form of swelling. The administration of drugs, especially steroids decreases or even fully suppresses said response in the cardiac tissue forming the device/tissue interface and its surroundings. An example for such steroids are glucocorticoids (also referred to as glucocorticosteroids) such as dexamethasone (DXA). Other examples may comprise different derivatives of dexamethasone, e.g., sodium dexamethasone phosphate or dexamethasone acetate.

The electrically insulating sub-assembly may be a single-piece element or may be composed of several members (i.e. a multi-piece element) which are fixed in a pre-defined position to each other, for example by press-fitting, welding and/or gluing. A modular design of the insulating sub-assembly is advantageous because the individual components can be manufactured easier and at lower cost compared to a single-piece design. The insulating sub-assembly may comprise, for example, a proximal end ring, a fixation member comprising the fixation means, for example tines or the like protruding in distal direction, a distal end ring and a member consisting of the medicament eluting depot. The fixation member may be clamped between the proximal end ring and the distal end ring. The members of the insulating sub-assembly that provide electrical insulation, e.g. a distal- and proximal end ring or the like, may comprise or may be fully composed of polyether ether ketone (PEEK), liquid crystal polymer (LCP), polysulfone (PSU) or other polymer material with similar properties. One or several members of the insulating sub-assembly may comprise elastic material which helps to establish a press-fitting connection between the members.

In one embodiment, said fixation means are or may comprise at least two fixation tines. One tine may be configured as a bent filament or wire-like element projecting/protruding from the insulating sub-assembly into distal direction. The tine may comprise a proximal abutting section and a distal flex zone. By being configured to penetrate into and, if applicable, out of the heart tissue when securing/anchoring the ID in a patient's heart, the tine provides the mechanical contact between the distal end of the ID and the patient's tissue. The tine may actively press the ID against the tissue, thereby ensuring good electrode-tissue contact that allows reliable pacing/sensing. The fixation means may comprise at least two tines, for example two tines, four tines or six tines, protruding from the distal end of the insulating sub-assembly, which provide fixation of the ID within the tissue of the patient at the desired treatment location after implantation. The fixation means, e.g. the at least one tine may partially or fully consist of biocompatible material, e.g. shape memory material, for example Nitinol.

The above problem is also solved by an implantable intracardiac device further comprising a housing, e.g. a cylindrical housing, and a header assembly as described above, wherein a feedthrough is accommodated at the distal end of the housing, wherein the proximal shaft section of the electrode of the header assembly protrudes through the feedthrough into the interior of the housing. Details about the housing are described above. The header assembly may be located distally from the feedthrough (through-going opening of the housing). The header assembly is fixed to the housing and hermetically seals the housing at its distal end.

In one embodiment of the implantable intracardiac device, the feedthrough is integrally formed with the housing or is formed by a separate feedthrough member which is fixed and hermetically sealed at the distal end face of the housing, for example by welding. In this embodiment, the feedthrough member comprises the through-going opening. The feedthrough may have a ring-like shape providing a receptacle similar to a female socket shape and, correspondingly, the insulating sub-assembly a male plug shape for correct and hermetically sealed accommodation of the insulating sub-assembly at the housing/feedthrough of the ID.

The present invention will now be described in further detail with reference to the accompanying schematic drawing, wherein
- Fig. 1: shows an embodiment of an inventive implantable ID with an inventive header assembly in a longitudinal-sectional view,
- Fig. 2: depicts schematically an enlarged section of the porous electrode head section of the embodiment of Fig. 1,
- Fig. 3: depicts the embodiment of Fig. 1 in the longitudinal-sectional view showing the drug elution profile, and
- Fig. 4: shows an enlarged longitudinal-sectional view of the electrode and the medicament eluting depot of the embodiment of Fig. 1.

In the following, the invention is described with regard to an ILP and a header assembly for an ILP, respectively. It may analogously be realized in another implantable intracardiac device or a respective header assembly, as well.

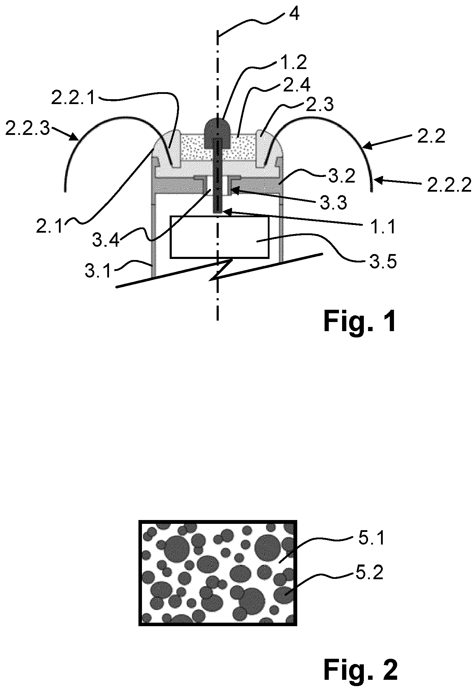

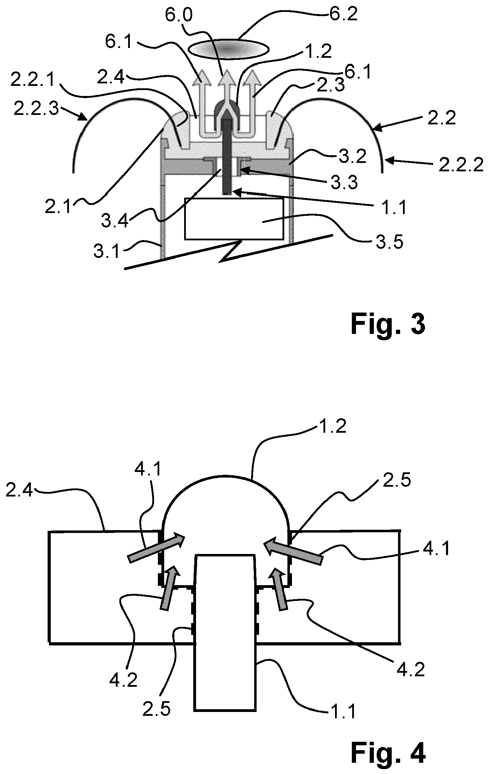

Fig. 1 and 3 show the components of an embodiment of the ILP with an embodiment of a header assembly. The header assembly comprises a pin-shaped electrode with a proximal shaft 1.1 and a porous distal head section 1.2, an electrically insulating sub-assembly comprising a ring-shaped proximal end ring 2.1, a fixation member 2.2 comprising a fixation ring 2.2.1, a first tine 2.2.2 and a second tine 2.2.3, a distal end ring 2.3, and a cylindrical medicament eluting depot 2.4 comprising a through hole 2.5. Further, a housing abuts the header assembly on its proximal side. It comprises a hollow cylindrical proximal section 3.1, a distal head section 3.2, a distal insert 3.3 and a feedthrough-insulating member 3.4 (electrically insulating), wherein the components distal head section 3.2, distal insert 3.3 and feedthrough-insulating member 3.4 are referred to as feedthrough of the ILP housing.

The medicament eluting depot 2.4 and the distal end ring 2.1 comprise the central through hole 2.5 for the electrode. Further, the feedthrough-insulating member 3.4 comprises a central through hole for accommodation of the electrode's proximal shaft section 1.1, as well. The though hole of the feedthrough and the through hole of the insulating sub-assembly are adjacently located along the longitudinal axis 4 and are concentric with regard to the longitudinal axis 4. The components of the insulating sub-assembly are axially symmetrical with regard to the longitudinal axis 4 defining the axial direction. The diameter of the central through hole of insulating sub-assembly and the feedthrough is such that the proximal shaft section 1.1 of the electrode is fixedly accommodated within these holes.

The distal head section 1.2 is electrically connected to the proximal shaft section 1.1. The proximal shaft section may extend into a respective proximal groove of the head section 1.2 (see Fig. 4). The head section 1.2 has a greater diameter than the shaft section 1.1. The proximal shaft section 1.1 has a cylindrical shape, whereas the distal head section 1.2 of the electrode has a combined cylindrical and hemispherical form and partly protrudes within the medicament eluting depot 2.4. The head section 1.2 consists of an 80 wt% platinum 20 wt% iridium alloy that was manufactured using a powder metallurgy sintering process, wherein the proximal shaft 1.1 may be mechanically fixed and electrically connected to the head section 1.2 during the sintering process. The microscopic structure of the head section 1.2 is illustrated in Fig. 2 showing connected metallic grains forming the matrix 5.1 and pores 5.2 that are interconnected providing the open porosity in the head section 1.2. The matrix 5.1 is electrically conducting. The open porosity is, for example, 0.05 - 0.75.

The through hole 2.5 of the insulating sub-assembly and the medicament eluting depot 2.4, respectively, has a first (proximal) section with a (smaller) first inner diameter and a (greater) second inner diameter at its distal end. The section of the through hole 2.5 extending within the medicament eluting depot 2.4 is depicted in Fig. 4 by means of a dashed line. The medicament eluting depot 2.4 forms a distal end face of the insulating sub-assembly that lies opposite to the transition face between the section of the through hole 2.5 with the smaller inner diameter and the section of the through hole 2.5 with the greater inner diameter. This distal end face and the inner surface of the through hole 2.5 of the section with the greater diameter abut the adjacent proximal end surface of the head section 1.2 of the electrode thereby providing an area where the medicament eluted from the medicament eluting depot 2.4 protrudes into the porous structure of the head section 1.2 (see arrows 4.2 in Fig. 4) and through it to the distal end of the head section 1.2. as it is shown in Fig. 3 by arrow 6.0. Further, the medicament eluting depot 2.4 forms a ring around the shell surface of the head section 1.2 of the electrode where medicament eluted from the medicament eluting depot 2.4 protrudes into the porous structure of the head section 1.2 of the electrode, as well (see arrows 4.1 in Fig. 4), and further through the distal head section 1.2 (see arrow 6.0 in Fig. 3). The distal end face of the medicament eluting depot 2.4 is further configured such that a section of this end face is exposed. From this exposed section of the medicament eluting depot 2.4 medicament is additionally eluted directly to the tissue of the patient if the ILP is anchored within the tissue. Arrows 6.1 depict the medicament elution directly from the medicament eluting depot 2.4. Both elution paths (see arrows 6.0 and 6.1) create a large area at the electrode/tissue interface (symbolized by area 6.2 in Fig. 3) to which the medicament is provided after implantation of the ILP at the target tissue location. The medicament eluting depot 2.4 consists of a silicone polymer matrix that is impregnated with a medicament, for example the steroid dexamethasone (DXA).

The distal end ring 2.3 has a central opening with a diameter that basically equals the outer diameter of the medicament eluting depot 2.4 so that the medicament eluting depot 2.4 is fixedly accommodated within the distal end ring 2.3. The top edge of 2.3 could cover part of the depot 2.4 (not shown) to better affix the depot to the header. The distal end ring 2.3 and the proximal end ring have corresponding conical surfaces extending in axial direction configured to clamp the fixation ring 2.2.1 comprising the fixation means thereby holding them in place. The distal end ring 2.3 and the proximal end ring consist of an electrically insulating and elastic material, for example PEEK.

The fixation ring 2.2.1 is, as mentioned above, clamped between two inwardly tapered sidewalls of the distal end ring 2.3 and the proximal end ring. The fixation ring 2.2.1 forms the base of the fixation member from which the bent first tine 2.2.2 and the bent second tine protrude in distal direction. Each tine 2.2.1 and 2.2.2 curves outwardly, that is away from the longitudinal axis 4 of the ILP.

The distal end of the distal end ring 2.3 does not form a blunt surface perpendicular to the longitudinal axis, but is rounded outwards in an arc-shape. The distal end of the proximal end ring 2.1 continues this shape, forming a rounded transition from the main housing to the device-tissue-interface. The proximal end ring 2.1 further comprises a radial groove on its outer perimeter. A circular inward protrusion of the housing's distal head section 3.2 locks into the radial groove and secures the proximal end ring 2.1 to the housing's distal head section 3.2. The proximal end ring 2.1 abuts the housing's distal head section 3.2 at an end face.

The housing's head section 3.2 consists of an annular disc which, as mentioned above, has a central through hole. The cylindrical proximal section 3.1 is hermetically closed at its proximal end (not shown) and encases the interior of the ILP such as a battery and an electronic module comprising a processor. These components are electrically connected to the proximal shaft section 1.1 of the electrode and provide the electrical stimulation of the heart and/or processing of electrical signals determined from the heart. Further, the housing may contain components for communication such as an antenna or inductive coil. The housing may consist of a titanium alloy or stainless steel. The housing's head section 3.2 accommodates a distal insert 3.3 within in its central through hole. The feedthrough-insulating member 3.4 accommodated within the through hole of the distal insert 3.3 provides electrical insulation of the electrode from the housing 3.1, 3.2. The proximal shaft section of the electrode protrudes through the central through hole of the feedthrough-insulating member 3.4.

The above ILP provides medicament elution at the same, comparatively large exact location where the sensing and pacing interactions occur effected by the porous electrode head section design as well as by its location of the abutting the medicament eluting depot 2.4. Additionally, accommodation of the medicament eluting depot within the insulating sub-assembly provides sufficient volume for the medicament so that it may be administered over a comparable long time period. Accordingly, the ILP with the above-described header assembly outlines a means to optimize the device/tissue interface and improves device performance.

## Claims

1. Implantable intracardiac device comprising a header assembly, wherein the header assembly comprises an electrically insulating sub-assembly (2.1, 2.2, 2.3, 2.4) and an electrode, wherein the electrode comprises a distal head section (1.2) and a proximal shaft section (1.1) and is accommodated within a through hole (2.5) of the insulating sub-assembly (2.1, 2.2, 2.3, 2.4), wherein the insulating sub-assembly (2.1, 2.2, 2.3, 2.4) comprises fixation means (2.2, 2.2.1, 2.2.2, 2.2.3) protruding from its distal end and a medicament eluting depot (2.4) forming at least one end face section of a distal end face of the insulating sub-assembly (2.1, 2.2, 2.3, 2.4) and/or at least one inner surface section of an inner surface of the through hole (2.5) of the insulating sub-assembly (2.1, 2.2, 2.3, 2.4), wherein the at least one end face section and/or the at least one inner surface section is located adjacent to and abuts the head section (1.2) of the electrode, wherein the head section (1.2) of the electrode comprises open porosity (5.2) within at least part of its volume configured to lead at least part of the medicament eluted from the medicament eluting depot (2.4) through the head section (1.2) and to the head section's distal end.

2. The implantable intracardiac device according to claim 1, wherein the medicament eluting depot (2.4) is doughnut-shaped and/or is at least partly exposed.

3. The implantable intracardiac device according to any one of the previous claims, wherein the head section (1.2) of the electrode comprises or consists of a material manufactured by a powder metallurgical sintering process.

4. The implantable intracardiac device according to any one of the previous claims, wherein the material of the head section (1.2) of the electrode comprises a platinum-iridium alloy comprising 70-92 wt% platinum and 8-30 wt% iridium, wherein the platinum and iridium alloy components together amount to 100 wt% at maximum.

5. The implantable intracardiac device according to any one of the previous claims, wherein the electrode head section (1.2) comprises a proximal end face that abuts a distal end face of the medicament eluting depot (2.4).

6. The implantable intracardiac device according to any one of the previous claims, wherein the electrode head section (1.2) has an exposed area at its distal end of 0.5 mm² to 2.5 mm².

7. The implantable intracardiac device according to any one of the previous claims, wherein said medicament eluting depot (2.4) is a medicament-impregnated polymer member.

8. The implantable intracardiac device according to any one of the previous claims, wherein said medicament comprises an anti-inflammatory drug.

9. The implantable intracardiac device according to any one of the previous claims, wherein said insulating sub-assembly is composed of several members (2.1, 2.2, 2.3, 2.4).

10. The implantable intracardiac device according to any one of the previous claims, wherein said fixation means (2.2) comprises at least two fixation tines (2.2.2, 2.2.3).

11. The implantable intracardiac device according to any one of the previous claims further comprising a housing (3.1, 3.2), wherein a feedthrough (3.2, 3.3, 3.4) is accommodated at the distal end of the housing, wherein the proximal shaft section (1.1) of the electrode of the header assembly protrudes through the feedthrough into the interior of the housing.

12. The implant of claim 11, wherein the feedthrough is integrally formed with the housing or is formed by a separate feedthrough member (3.4) which is fixed and hermetically sealed at the distal end face of the housing.
